# EUROPEAN PATENT SPECIFICATION

(11) **EP 2 836 612 B1**
(45) Date of publication and mention of the grant of the patent: **01.05.2019**
(21) Application number: 12706932.6
(22) Date of filing: 15.02.2012
(51) Int. Cl.: C12N 1/20, A01N 63/02, C12R 1/39

(54) **NOVEL PSEUDOMONAS FLUORESCENS STRAIN AND USES THEREOF IN THE BIOLOGICAL CONTROL OF BACTERIAL OR FUNGAL DISEASES**
NEUARTIGER PSEUDOMONAS FLUORESCENS STAMM UND VERWENDUNGEN DAVON BEI DER BIOLOGISCHEN BEKÄMPFUNG VON BAKTERIEN- ODER PILZERKRANKUNGEN
NOUVELLE SOUCHE PSEUDOMONAS FLUORESCENS ET SON UTILISATION DANS LA LUTTE BIOLOGIQUE CONTRE LES MALADIES BACTÉRIENNES OU CRYPTOGAMIQUES

(43) Date of publication of application: 18.02.2015
(73) Proprietor: Alma Mater Studiorum - Università di Bologna, 40126 Bologna (IT)
(72) Inventor: BAZZI, Carlo, I-40042 Lizzano in Belvedere BO (IT); BIONDI, Enrico, I-47121 Forlì FC (IT)
(74) Representative: Predazzi, Valentina
(86) International application number: PCT/IB2012/050694
(87) International publication number: WO 2013/121248

(56) References cited:
- WO-A1-2010/037072
- M.A.A Seleim ET AL: "biological control of bacterial wilt of tomato by plant growth promoting rhizobacteria", plant pathology journal, 1 January 2011 (2011-01-01), pages 146-153, XP55039810, Retrieved from the Internet: URL:http://docsdrive.com/pdfs/ansinet/ppj/ 2011/146-153.pdf [retrieved on 2012-10-02]
- N. DANDURISHVILI ET AL: "Broad-range antagonistic rhizobacteria Pseudomonas fluorescens and Serratia plymuthica suppress Agrobacterium crown gall tumours on tomato plants", JOURNAL OF APPLIED MICROBIOLOGY, vol. 110, no. 1, 1 January 2011 (2011-01-01), pages 341-352, XP55039803, ISSN: 1364-5072, DOI: 10.1111/j.1365-2672.2010.04891.x
- Jordi Cabrefiga ET AL: "Mechanisms of antagonism of Pseudomonas fluorescens EPS62e against Erwinia amylovora, the causal agent of fire blight", INTERNATIONAL MICROBIOLOGY, 1 January 2007 (2007-01-01), pages 123-132, XP55039801, DOI: 10.2436/20.1501.01.18 Retrieved from the Internet: URL:http://revistes.iec.cat/index.php/IM/a rticle/viewFile/4c457ca1c1a67.002/9610 [retrieved on 2012-10-02]

## Description

The present invention relates to a novel bacterial strain as biological control agent of diseases caused by pathogens such as bacteria or fungi, such as plant diseases, to compositions comprising the same, to methods for treatment and/or prevention of plant diseases, i.e. management of plant diseases, comprising the management of the bacterium or of the composition herein disclosed.

### STATE OF THE ART

Fungal and bacterial plant pathogens are among the most important factors that cause serious losses to several crops every year.

Their attacks occur before or after harvesting and cause significant losses not only to the agricultural compartment but also to the whole production line of products in all the technical fields related to the use of plant materials.

Usually, plant diseases are controlled with the application of chemical products or mixtures containing active principles having antibacterial/antifungal activity; however, the routine use of such molecules (e.g. antibiotics) has various known undesirable side effects such as developement pesticide-resistance, poisoning of herbivores (e.g. bees, mammalians, etc.), damages to biodiversity, accumulation in the soil and in the waters of undesired chemicals.

A biological control of plant diseases represents a desirable alternative approach that would limit the use of harmful pesticides in integrated control programmes. In plant pathology, the term biocontrol indicates the use of microbial antagonists to prevent or treat the onset and the development of the infectious process. Different mode of actions of biocontrol-active microorganisms in controlling plant diseases include hyper parasitism, antibiosis, cross protection, competition for penetration sites and nutrients, and induced resistance. There are various methods known in the art for the application of biocontrol agents: a direct spray application on susceptible plant organs at a high population level of said agents, or a localized application on the plant for a systemic activity against pathogen populations. The development of techniques for the biological control of plant diseases and of the large scale use of beneficial microorganisms has been slow, mainly due to the variable performances of said microorganisms under different field conditions. In order to overcome this problem, it is important to develop new biocontrol formulations having a higher level of stability and survival in space and time. A significant improvement has been made in different aspects of the biological control of fungal plant diseases, but this area still needs further investigations to face and solve the existing problems. By way of example, fire blight by *Erwinia amylovora* is a destructive bacterial disease of pomaceous species such as apple and pear. Heavy *E. amylovora* infections kill blossoms, shoots, limbs, and even the whole tree. In general, economic losses are great considering that this disease is not easily and effectively controlled by conventional agronomic (removal and destruction of infected plant organs, use of resistant rootstocks and cultivars, etc.) and chemical approaches (copper and other compound sprays). At bloom (i.e. when allowed by law in several countries), antibiotic sprays can be highly effective not only against the blossom blight phase of the disease but also in preventing the development and spread of the other blight phases. However, strains of the pathogen that are resistant to the antibiotic streptomycin are present in some orchards in the eastern U.S., and are widespread in most apple and pear growing regions of the western U.S. Biological control agents, although not widely used, have provided partial control of blossom blight infections, even if more effective biological agents are required for a large scale commercial use.

In order to have more effective biological control strategies in the future, further research is crucial to deeper investigate over some unrevealed aspects of biocontrol. This research includes developing novel formulations, understanding the impact of environmental factors on biocontrol agents, mass production of biocontrol microorganisms and the use of biotechnology and nano-technology in the improvement of biocontrol mechanisms and strategies. Future outlooks of biocontrol of plant diseases is bright and promising and with the growing demand for biocontrol products among the growers, it will be possible to use the biological control as an effective strategy to manage plant diseases, grow healthy crops, increase yields with minimal impacts on the surrounding environment, and approach a sustainable agricultural system.

However, before biocontrol can become the elected way for fighting plant diseases, the scientist have to identify suitable Biological Control Agents (BCA) that will allow fighting specific diseases.

WO 2010/037072 discloses a fluorescent *Pseudomonas* bacterium with activity against *Ralstonia, Xanthomonas, Pseudomonas* and *Erwinia.*

M.A.A Seleim et al, plant pathology journal, 1 January 2011, pages 146-15, discloses *Pseudomonas fluorescens* with activity against *Ralstonia solanocearum.*

N. Dandurishvili et al, Journal of Applied Microbiology, vol. 110, no. 1, 1 January 2011, pages 341-352 discloses *Pseudomonas fluorescens* with activity against *Agrobacterium vitis.*

Jordi Cabrefiga et al, International Microbiology, 1 January 2007, pages 123-132 disclsoses *Pseudomonas fluorescens* with activity against *Erwinia amylovora.*

From the data available in the art, it appears that selected strains of *Pseudomonas fluorescens* (Pf) can be considered as possible candidates for use against various plant diseases of different aetiology, however, up to date, there are no experimental data that allow a complete knowledge on their capability of colonisation of plant organs and of surviving thereon for a long time with high population levels and to adapt to the environmental variability that exists in the fields, nor there are clear protocols on how to select appropriate strains.

The only clear indication is that, the suitable candidate as BCA must be non toxic for plants and humans and also capable of preventing/treating plant infections in the glass house and in the field, and preferably being active against various pathogens.

### DESCRIPTION

The present invention discloses a *Pseudomonas fluorescens* (also indicated as *P. fluorescens*) bacterium having the antipathogenic activities (as disclosed in the detailed description of the invention) of the *P. fluorescens* having the deposit number DSM 25556 (deposited under the Budapest treaty at DSMZ on Jan 13 2012), for the prevention and/or treatment of plant diseases of bacterial or fungal aetiology.

The bacterium of the invention being characterised in that it is at least capable of preventing the onset of the infection on plants by various phytobacteria such as *Xanthomonas arboricola* pv. *pruni, Ralstonia solanacearum, Erwinia amylovora, Pseudomonas syringae* pv *syringae, Pseudomonas syringae* pv *mors-prunorum, Pseudomonas syringae pv. actinidiae, Pseudomonas syringae pv. persicae; Pseudomonas viridiflava, Agrobacterium vitis, Pectobacterium carotovorum* subsp. *carotovorum;* and by fungal plant pathogens such as *Fusarium oxysporum* f. spp.

The invention also discloses compositions comprising said bacterium, a method of treatment of plants comprising putting in contact said plant or a part thereof with suitable amounts of the living bacterium or of the composition of the invention, a method of treatment of plants comprising putting in contact a living vector, such as an insect, with suitable amounts of said living bacterium, wherein said vector by contacting the plant or a part thereof, will allow the colonisation of said plant or part thereof by said bacterium.

The bacterium of the invention has been isolated and cultured by the inventors and its activity in the biocontrol of various plant diseases has been verified both *in vitro* and *in planta* in the glasshouse and in the field.

The bacterial strain (i.e. the bacterium) of the invention has shown to be particularly active on infections caused by the aetiological bacteria or fungi listed above as it is a strong antagonist of the same and, when present on the plant or part of the plant of interest, competes with the growth of the pathogen.

In one of its embodiments, the invention hence teaches the use of a natural organism as herein described for treating or preventing a dangerous plant disease/s without using GMO material or frequent applications of undesired pesticides. The bacterium of the invention can be used as an alternative and/or integration to pesticides in the prevention and/or treatment of infections caused by phytopathogenic bacteria and fungi in the laboratory, in the glasshouse and in the field.

The bacterium of the invention can also be combined with further active and/or inert compounds that allow a better growth and/or a better colonisation and/or a longer viability.

### DETAILED DESCRIPTION OF THE FIGURES

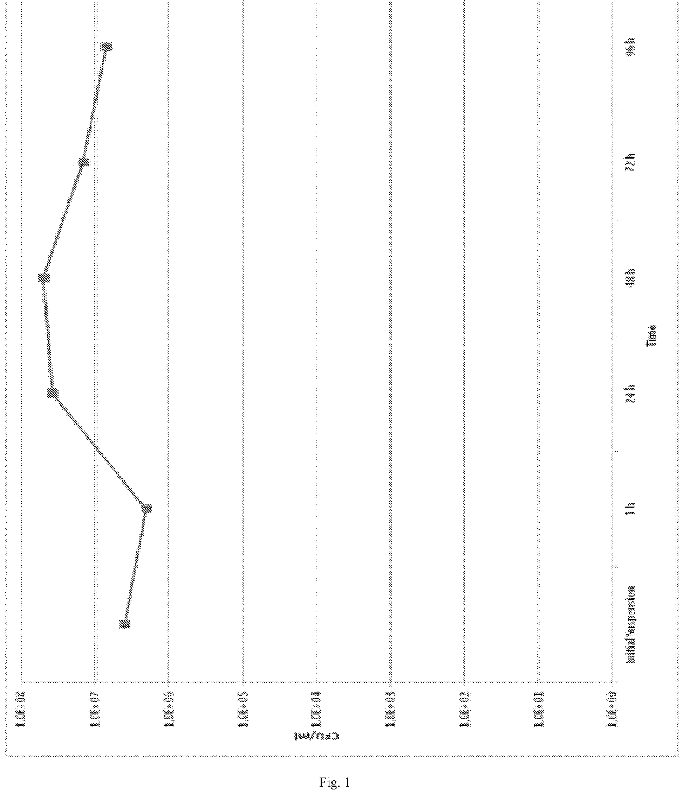

Figure 1 shows the colonisation by the BB20 bacterium having deposit number DSM 25556 (also referred as BB20 in the present application) in female flowers of *Actinidia chinensis* cv. HORT16 (Yellow kiwi). The graph shows a high CFU value maintained for a period of at least 96 hours on the flower.

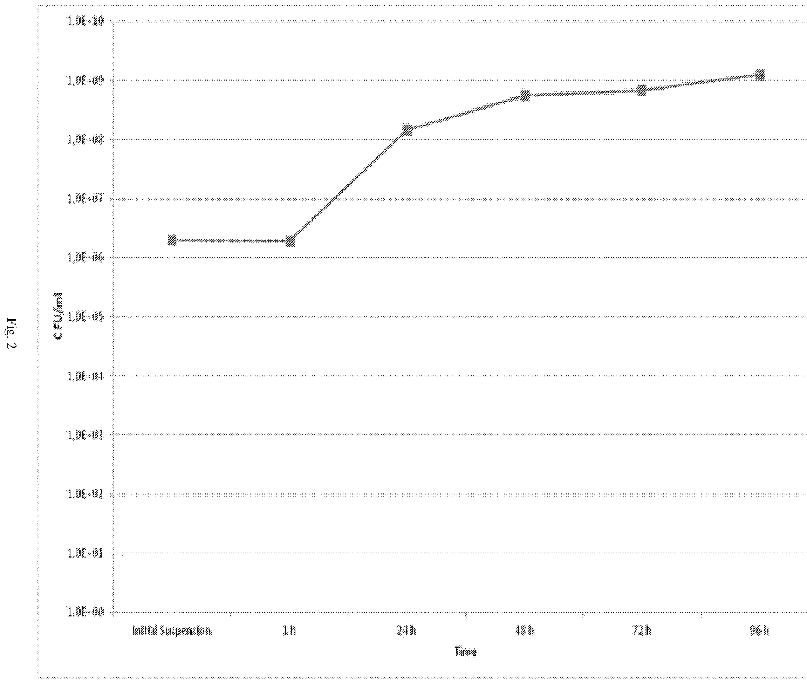

Figure 2 shows the colonisation by the BB20 bacterium having deposit number DSM 25556 in female flowers of *Actinidia deliciosa* cv. Hayward (Green kiwi). The graph shows a high CFU value maintained for a period of at least 96 hours on the flower.

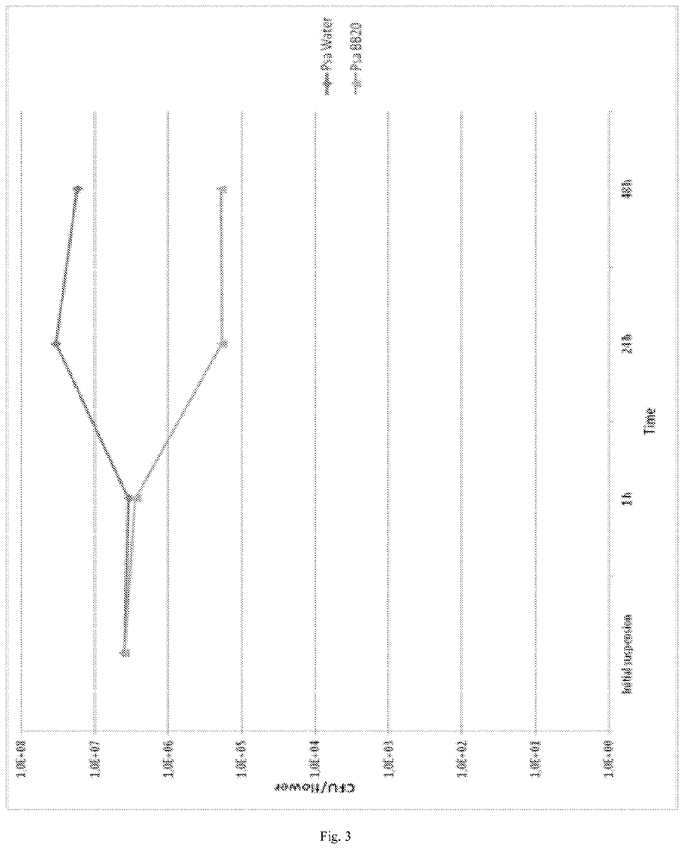

Figure 3 shows the antagonistic activity of the BB20 bacterium having deposit number DSM 25556 in female flowers of *A. chinensis* cv. HORT16 (Yellow kiwi) versus *P. syringae* pv. *actinidiae* strain (Psa) CRA-FRU 3.1. The initial bacterial CFU/flower is the same for both strains and dramatically drops for CRA-FRU 3.1 when antagonised by BB20.

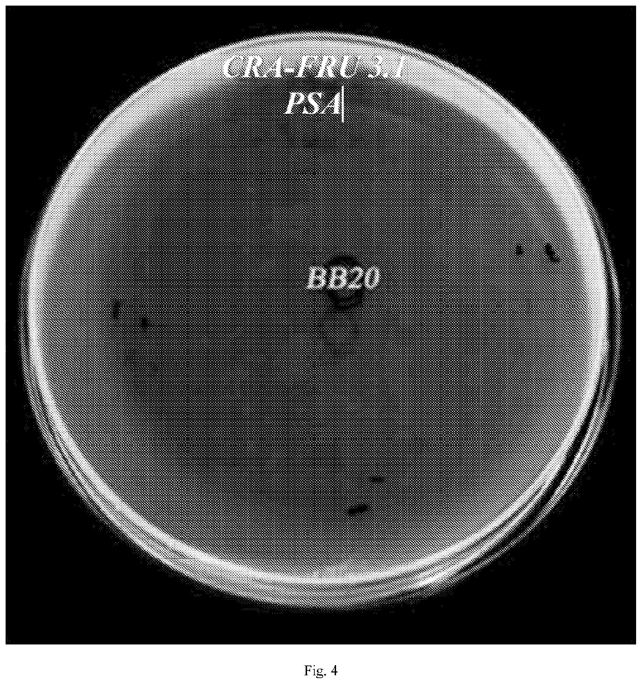

Figure 4 shows the *in vitro* inhibition of CRA-FRU 3.1 of *P. syringae* pv. *actinidiae* by the BB20 bacterium having deposit number DSM 25556. The inhibition halo covers almost the whole surface of the plate.
Figure 5 shows typical BOX-, REP- and ERIC-PCR patterns of the the BB20 bacterium having deposit number DSM 2555 (arrow) compared with those of different *P*. *fluorescens* strains.

### DETAILED DESCRIPTION OF THE INVENTION

The present invention discloses a *P. fluorescens* bacterium as defined in detail below, (also represented in a non limiting way by the bacterium herein defined as "BB20" having the deposit number under the Budapest treaty DSM 25556), for the prevention of plant diseases of bacterial and fungal aetiology.

In one embodiment, the bacterium of the invention is a *P. fluorescens* biovar 1 bacterium having all of the antipathogenic activities of the *P*. *fluorescens* that was deposited on Jan 13 2012 at DSMZ under the Budapest treaty, deposit number DSM 25556, wherein said antipathogenic activities comprise at least the following:
presence of *in vitro* inhibitory activity on solid medium at least against: *Xanthomonas arboricola* pv. *pruni, Ralstonia solanacearum, Erwinia amylovora, Pseudomonas syringae* pv *syringae, Pseudomonas syringae* pv *morsprunorum, Pseudomonas syringae pv. actinidiae, Pseudomonas syringae pv. persicae; Pseudomonas viridiflava, Agrobacterium vitis. Pectobacterium carotovorum* subsp. *carotovorum;* and
presence of *in vitro* inhibitory activity on solid medium of the miceliar development of the fungus *Fusarium oxysporum* f. spp.

According to the invention said inhibitory activity may be assayed by standard assays such as, by a non limiting way of example, the antibiotic production assay described by Vanneste, J.L., Yu, J. and Beer, S.V. 1992 "Role of antibiotic production by Erwinia herbicola Eh252 in biological control of Erwinia amylovora" Journal of Bacteriology 174: 2785-2796 (said antibiotic production assay being described in detail at page 2786 in the paragraph Materials and Methods of the cited document); and/or the antifungal assay described by De Boer M, lentse van der Sluis, Leendert C. van Loon and A.H.M. Baker, 1999 "Combining fluorescent Pseudomonas spp. strains to enhance suppression of Fusarium wilt of radish" European Journal of Plant Pathology 105: 201-210, 1999 (the antifungal assay being described in detail at page 203, paragraph Materials and Methods of the document cited).

The bacterium of the invention will also disclose at least one of the above antipathogenic activities *in planta,* said antipathogenic activities being assayable with any known method such as described by Alexandrova M., C. Bazzi and P. Lameri, 2002" Bacillus subtilis strain BS-F3: colonisation of pear organs and its action as a biocontrol agent" Acta Horticulturae 590: 291-297"; (the assays *in planta* being described in detail at page 292 in the paragraph Materials and Methods of the cited document) or by Bazzi C., M. Alexandrova, E. Stefani, F. Anaclerio and T.J. Burr, 1999 "Biological control of Agrobacterium vitis using non-tumorigenic agrobacteria" Vitis 38: 31-35 (the assays being described in detail at page 32 in the paragraph Materials and Methods of the cited document).

In an embodiment of the invention the bacterium is the *Pseudomonas fluorescens* biovar 1 having deposit number under the Budapest treaty DSM 25556 (also defined as BB20 in the present description).

This bacterium is characterised by the presence of an *in vitro* inhibitory activity on solid medium at least against: *Xanthomonas arboricola* pv. *pruni, Ralstonia solanacearum, Erwinia amylovora, Pseudomonas syringae* pv *syringae, Pseudomonas syringae* pv *morsprunorum, Pseudomonas syringae pv. actinidiae, Pseudomonas syringae pv. persicae, Pseudomonas viridiflava, Agrobacterium vitis, Pectobacterium carotovorum* subsp. *carotovorum* and by the presence of an *in vitro* inhibitory activity on solid medium at least against the miceliar development of the fungus *Fusarium oxysporum* f. spp.

In a further embodiment, the *P. fluorescens* bacterium of the invention is a *P. fluorescens* deriving from the bacterium having the deposit number under the Budapest treaty DSM 25556 wherein said derived bacterium (also referred as deriving bacterium) is characterised in that it maintains all of the antipathogen/inibitory activities of the deposited strain DSM 25556. By "deriving or derived bacterium" it is herein intended any bacterium obtainable by natural or induced mutation, crossing, variation of the genetic information by molecular genetics technology of the *P. fluorescens* biovar 1 having deposit number DSM 25556 maintaing all of the inhibitory acrtivities agains pathogens of DSM 25556, by way of example, the derived bacterium can be a bacterium having deposit number DSM 25556 transformed with a vector carrying one or more genes for antibiotic resistance or mutated for antibiotic resistance so to allow co-administration of the BCA and antibiotics for the treatment or the prevention of the diseases according to the invention.

It will be understood by the skilled person that the derived bacterium, will also obviously share several genetic markers with the related bacterium having deposit number DSM 25556.

Further specific features characterising the strain having deposit number DSM 25556 are herein provided.

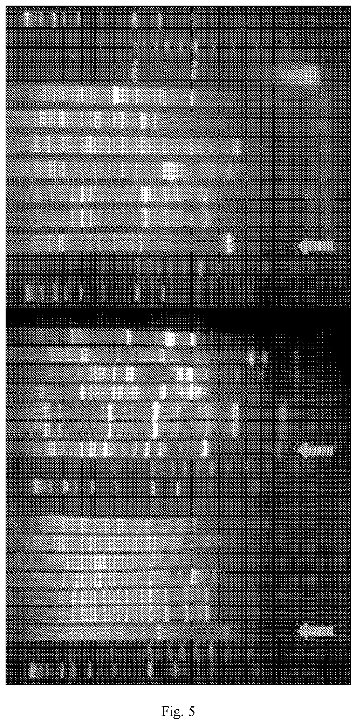

The bacterium having deposit number DSM 25556 has been isolated from apple cv. Oregon spur buds, it is a Gram-negative bacterium belonging to the *P. fluorescens* biovar 1 and has the following phenotypical/biochemical footprints:
fluorescent pigments are present,
the levansucrase enzyme is present,
pectinolytic enzymes are absent,
the cytochrome C oxidase enzyme is present,
the enzyme arginine dihydrolase is present,
no nitrate reduction activity is present,
it grows at 4°C but not at 41°C
"BB20" has typical BOX-, REP- and ERIC-PCR patterns, distinguishable from other bacterial strains belonging to *P. fluorescens* (Fig. 5). (see: Versalovic J., M. Schneider, F.J. de Bruijn and J.R. Lupski. 1994 - Genomic fingerprinting of bacteria using repetitive sequence-based polymerase chain reaction Meth. Mol. Cell. Biol., 5: 25-40).

Moreover, the bacterium according to the invention does not induce an hypersensitive reaction in tobacco leaf panels (HR-negative),
does not provoke the lysis of parenchymal tissues in potato tubers (Potato Rot-negative) and does not provoke the lysis of parenchymal tissues in bean seeds.

The BCA of the invention is hence a non-phytopatogenic strain and its application on plants organs (flowers, fruits, roots) of various plants such as pear, apple, plum, actinidia and tomato, showed that the bacterium is also not toxic for plants.

Furthermore, the bacterium of the invention does not produce 2, 4-DAPG nor PCA.

Moreover, as already stated, it has also an *in vitro* inhibitory activity (e.g. on solid medium assays) at least against: *Xanthomonas arboricola* pv. *pruni, Ralstonia solanacearum, Erwinia amylovora, Pseudomonas syringae* pv *syringae, Pseudomonas syringae* pv *morsprunorum, Pseudomonas syringae pv. actinidiae, Pseudomonas syringae pv. Persicae, Pseudomonas viridiflava, Agrobacterium vitis, Pectobacterium carotovorum* subsp. *carotovorum,* and inhibits the miceliar development of the fungus *Fusarium oxysporum* f. spp.

As indicated above, several assays can be used for testing the inhibitory activities listed above, e.g. as in Vanneste, J.L., Yu, J. and Beer, S.V. 1992 "Role of antibiotic production by Erwinia herbicola Eh252 in biological control of Erwinia amylovora" Journal of Bacteriology 174: 2785-2796 see page 2786 in the paragraph Materials and Methods; De Boer M, lentse van der Sluis, Leendert C. van Loon and A.H.M. Baker, 1999 "Combining fluorescent Pseudomonas spp. strains to enhance suppression of Fusarium wilt of radish" European Journal of Plant Pathology. 105: 201-210, 1999 see page 203, paragraph Materials and Methods.

The same assays can also be used to verify that at least one of the said activities has been maintained by the derived bacterium according to the present description.

The bacterium of the invention (including the derived bacterium) shows also a verified *in planta* inhibitory activity on plant parts such as flowers, fruits and roots, against *Erwinia amylovora, Xanthomonas arboricola* pv. *pruni, Ralstonia solanacearum.*

Given the data above, the skilled person will be able to identify strains of the bacterium of the invention also from *P. fluorescens* cultures and will not necessarily need to use the deposited strain which is just an example of the bacterium of the invention and is not a limitation.

The term biological control and its abbreviated synonym "biocontrol" according to the description applies to the use of microbial antagonists to suppress diseases, as well as the use of host-specific pathogen to control weed populations, the organism suppressing the pest or pathogen being here also referred to as the Biological Control Agent (BCA).
The BCA of the invention is hence a *P. fluorescens* biovar 1 bacterium as defined above. In an embodiment of the invention, the plant diseases of bacterial or fungal aetiology can be selected respectively from one or diseases caused by *Xanthomonas spp., Erwinia spp., Pseudomonas spp., Ralstonia spp., Agrobacterium spp., Pectobacterium spp., Fusarium spp.*

In an embodiment of the invention, the disease is a plant disease caused by *Xanthomonas arboricola* pv. *pruni, Ralstonia solanacearum, Erwinia amylovora, Pseudomonas syringae* pv *syringae, Pseudomonas syringae* pv. *morsprunorum, Pseudomonas syringae pv. actinidiae, Pseudomonas syringae pv. persicae; Pseudomonas viridiflava, Agrobacterium vitis, Pectobacterium carotovorum* subsp. *carotovorum; Fusarium oxysporum* f. spp. infections.

In the meaning of the invention one of the plant diseases of bacterial or fungal aetiology is caused by *Xanthomonas arboricola* pv. *pruni* (Smith) Vauterin et al. also known as *Xanthomonas campestris* pv. *pruni* (Smith) Dye, *Xanthomonas pruni* (Smith) Dowson having the taxonomic position: Bacteria: Gracilicutes and being a quarantine pathogen included in the EPPO A2 list 2011.

The common names of the disease caused by *Xanthomona arboricola* pv. *pruni* are: Bacterial leaf spot/bacterial canker, shot-hole, black spot (English).

The skilled person will know which plants are attacked and by *Xanthomona arboricola* pv. *pruni* and hence can be treated and/or protected against *Xanthomona arboricola* pv. *pruni* infections.

By way of example, it is known that *Xanthomona arboricola* pv. *pruni* attacks *Prunus* spp., and particularly the fruit crops almonds, peaches, cherries, plums, apricots and *P. salicina.* Cultivars of the Sino-Japanese group (*P. japonica* and *P. salicina*) are generally more susceptible than European plums. Other exotic or ornamental species of *Prunus* attacked include *P*. *davidiana* and *P. laurocerasus.*

Due to the very wide distribution of the pathogenic bacterium (at least Europe, Asia, Africa, North America, South America and Oceania) and the economic impact caused by heavy infections, the skilled person will appreciate the usefulness of the Biological Control Agent (i.e. *Pseudomonas fluorescens* bacterium (BB20) deposit number under the Budapest treaty DSM 25556) of the present invention on the light of the present disclosure.

In the meaning of the invention one of the plant diseases of bacterial or fungal aetiology is caused by *Ralstonia solanacearum.* Commonly known synonyms of this bacterium are: *Bacterium solanacearum* (Smith) Chester, *Burkholderia solanacearum* (Smith) Yabuuchi et al. (1992), *Pseudomonas solanacearum* (Smith) Smith.

The bacterium has the following taxonomic position: Bacteria: Gracilicutes and it is a quarantine pathogen included in the EPPO A2 2011 list behaving as a complex of variants, variously described as groups, races, biovars, biotypes, sub-races and strains.

The common names of the disease caused by *R solanacearum* are brown rot (potato), southern bacterial wilt (tomato) Moko disease (banana), Granville wilt (tobacco) (English).

*R solanacearum* has an extremely wide host range and is the causal agent of various devastating bacterial wilt diseases. Over 200 species, especially tropical and sub-tropical crops are susceptible to one or other of the variants of the pathogen. Worldwide, the most important crops are: tomatoes, *Musa* spp. tobacco and potatoes. Some minor host crops are: *Anthurium* spp., groundnuts, *Capsicum annuum,* cotton, rubber, cassava, castor beans, aubergines and ginger. Many weeds (e.g.: *Solanum dulcamara*) are also hosts of the pathogen and therefore increase the potential of *R solanacearum* to build up inoculums. Normally the pathogens infect plants passing through injured roots, stems wounds or through stomata moving in the vascular bundles and colonizing the xylem with four major steps: appearance of root tip symptoms, root tip cortical cell invasion, vessels colonization, and foliar wilting.

Very few phytopathogenic bacteria are able to colonize intact plant roots as *R. solanacearum,* even if the local soil provides a favourable environment for bacterial growth. *R. solanacearum,* a Gram-negative soil borne pathogen responsible for bacterial wilt, naturally invades plants through axils of secondary roots. *R. solanacearum* attaches to the root, finds nutrients, multiplies, migrates into plant tissues and penetrates the xylem thus causing the disease. The main path for International spread of Race 3 of this pathogen is by (latently) infected seed potatoes and other vegetative propagating materials; tubers with latent infection are the most probable means of introduction into a new area.

The high incidence of plant mortality and the lack of effective control methods make *R. solanacearum* one of the world's most destructive plant pathogens. In one embodiment of the invention, e.g. in the case of *R. solanacearum* infections, the BCA or the composition of the invention can be directly applied on propagation materials (seeds, tubers, etc..), in soils and on the root apparatus of plantlets before transplanting.

In the meaning of the invention one of the plant diseases of bacterial or fungal aetiology is caused by *Agrobacterium vitis* Ophel and Kerr (1990); synonym: *Rhizobium vitis* Young et al. (2001) having the following taxonomic position: Bacteria: Gracilicutes.

Although in the EU the bacterium is not listed as a quarantine pest it's commonly regarded as a harmful, widespread pathogen ("quality organism") that can reduce the value of propagation materials. Furthermore, in other Countries it is considered a quarantine pest (zero tolerance). In International trade, *A. vitis* may be included among the so-called "regulated non quarantine pests" (Int. standard of Phytosanitary Measures, ISPM 16-FAO).

The common names of the disease caused by *A. vitis* are: grapevine crown gall (English); tumore batterico/rogna della vite (Italian).

The natural host range of *A. vitis* is restricted to grapevine. Cultivars of the European grapevine (*Vitis vinifera*) are more susceptible than other *Vitis* spp. and interspecies hybrids. The disease occurs throughout the world where grapes are grown and can lead to vine decline and mortality in vineyards, especially those subject to freezing injuries which provide an important site where the disease can initiate. The crown gall pathogen survives systemically within the grapevine and causes disease (tumours) at wounded areas on the vine. *A. vitis* also causes areas of root necrosis on infected vines.

Crown gall represents a unique biological system (interkingdom transfer): the export of genetic material from a bacterium to a plant with a stable integration of bacterial oncogenes in the plant cell genome (tumour transformation).

The pathogen may be present in asymptomatic propagation materials raising the problem of the marketing of latently infected materials, acting as means of short and long distance dissemination of tumourgenic agrobacteria.

The treatment of the propagation with the bacterium or with the composition of the invention can assist in the prevention of the spread of the disease through propagating material.; by way of example, the BCA or the composition of the invention can be used for a root dip before planting of propagation materials (bio film formation as a Plant Growth Promoting Rhizobacterium, PGPR), a vine injection or annual spray.

In the meaning of the invention one of the diseases of bacterial or fungal aetiology is caused by *Erwinia spp,* which is a well known anarerobial bacterial species causing diseases in plants, all known diseases caused by all known pathogenic *Erwinia* strains such as *E. amylovora, E. carotovora, E. chrysantemi* etc. are part of the invention.

In an embodiment of the invention the plant disease may be caused by *E. amylovora* (Burrill) Winslow et al. also known as *Micrococcus amylovorus* Burrill, *Bacillus amylovorus* (Burrill) Trevisan, *Bacterium amylovorus* (Burrill) Chester, *Erwinia amylovora* (Burrill) Winslow et al. f.sp. *rubi* Starr et al. having the following taxonomic position: Bacteria: Gracilicutes and being a quarantine pest included in the EPPO A2 list, its introduction being prohibited by almost countries (see phytosanitary measures).

The common name of the disease caused by *Erwinia amylovora* is fire blight.

The principal and most susceptible hosts are in the sub-family *Pomoideae* of the family *Rosaceae.* The following plants are considered as important hosts, from both economic and epidemiological points of view: *Amelanchier alnifolia, A. canadensis, Malus* spp., *Chaenomeles* spp., *Cotoneaster* spp., *Crataegus* spp., *Cydonia* spp., loquats, medlars, *Pyrus* spp., *Pyracantha* spp., *Sorbus* spp., *Stranvaesia davidiana.* Of these, the most susceptible are: *Crataegus* (most species), *Cydonia* (most species), *Cotoneaster bullatus, C. dammeri* (except cv. Eichholz No. 1), *C*. *lacteus, C. lucidus, C. microphyllus, C. moupinensis, C. salicifolius, C. watereri,* pear cultivars Alexandrine Douillard, Durondeau and Passe Crassane, apple cultivars Idared, Red Jade, Van Eseltine, *Pyracantha fortuneana* cv. Orange Glow, *Sorbus aria* and *Stranvaesia davidiana.* The following are not, however, considered as hosts: *Crataegus arnoldiana, C. phaenopyrum, C. viridis, Sorbus intermedia.*

*Crateagus* and *Pyrus amygdaliformis* play an important role as sources of inoculums in northern Europe and the Mediterranean, respectively, because of their abundance in these areas. *Crateagus* is widely used as a wayside and hedging plant, while *P. amygdaliformis* is a common wild plant of the Mediterranean landscape.

Fire blight is a destructive disease and is a major threat for the cultivated apples and pears as well as for the nursery trade. The phytosanitary risk is high, due to the capacity of the pathogen to live in asymptomatic plant organs. The economic impact of the disease is unpredictable and after favourable weather conditions yield is considerably reduced and in some cases nullified. It is often necessary to cut and burn the heavily infected trees.

The hosts can be infected by the pathogen that penetrates through natural openings and wounds: typical symptoms are wilt and death of flower clusters, twig and shoot necrosis forming the so-called "shepherd's crook", leaf, fruit, limb and trunk blight. The progression of the infection, associated to the movement of the bacteria within plant tissues, leads to the formation of typical bark cankers, surrounded or not by irregular cracks. Hold-over cankers, as well as all the other infected organs, may act as important sources of inoculums (ooze evasion) during the growing season.

Eradication measures have proved ineffective to prevent the spread of the disease. Strict phytosanitary measures on imported host plant material and vigilance in orchards and nurseries are necessary to prevent or delay the spread of *E. amylovora* into not infested areas.

Disease control is quite difficult, especially where the use of antibiotics is prohibited. In the last few years, new strategies of integrated control have been developed, based on the use of complex molecules of biotic (e.g. bacterial proteins) or abiotic (prohexadione-Ca, acybenzolar-S-methyl, salicilic acid, phosethyl-AI) origin which trigger natural plant defence responses (also defined herein as "elicitors of plant defence responses").

According to the invention, the BCA or the composition comprising the same may be used for annual sprays to prevent flower and shoot infections. The use of pollinating insects (*Apis mellifera* and *Osmia cornuta*) has a potential as carrier of antagonist bacteria in biological control of fire blight. The BCA of the present invention can be used in integrated applications in combination with elicitors of disease resistance responses such as the ones indicated above.

In the meaning of the invention a further plant disease of bacterial or fungal aetiology can be a disease caused by *Pseudomonas spp.,* such as *P*. *aeruginosa, P. oryzihabitans, and P. plecoglossicida, P. putida, P syringae, P. solanacearum,* etc.

In an embodiment of the invention the disease will be caused by *P. syringae* pv. *actinidiae* Takikawa et al. having the following taxonomic position: Bacteria: Gracilicutes; the common name of the disease caused by this Gram- bacterium being Bacterial canker of Kiwifruit (English).

The disease has been reported in Europe (EPPO Region, Alert List), Asia, South America, Oceania and affects the *Actinidia* species: *A. deliciosa, A. chinensis, A. arguta,* and *A. kolomikta* (there is no data on the susceptibility of other *Actinidia* species). The pathogen survives in the infected tissues and in spring it evades as ooze, penetrating though stomata, lenticels and wounds. The development of the infection is rapid and systemic.

The pathogen may be present in asymptomatic propagation materials raising the problem of the marketing of latently infected materials. The effectiveness of copper sprays after pruning, after hail storms and during leaf fall is limited. The BCA or the composition can be used on propagating material as described above.

In the meaning of the invention one of the plant diseases of bacterial or fungal aetiology is caused by *Pseudomonas syringae* pv. *syringae* van Hall having the following taxonomic position: Bacteria: Gracilicutes the common name of the disease caused by this Gram-bacterium being "Disseccamento rameale dell'actinidia (Italian)" e.g. actinidia branches withering.

Typical symptoms of the disease are cortical lesions, wilting of canes, bud necrosis and necrotic leaf spots. The pathogen may be present in asymptomatic propagation materials raising the problem of the marketing of latently infected materials. Also in this case the effectiveness of early copper sprays after pruning, after hail storms and during leaf fall is limited.

As above, the BCA or the composition of the invention is useful also for the treatment of propagating material.

In the meaning of the invention one of the plant diseases of bacterial or fungal aetiology is caused by *Pseudomonas viridiflava* (Burkholder) Dowson having the following taxonomic position: Bacteria: Gracilicutes, the common name of the disease caused by this Gram-bacterium being Bacterial spot (English).

Typical symptoms are necrotic spots and browning of leaf and blossom tissues. An internal rot may damage blossom buds. No lesions on canes and trunks are visible. Partial floral infections may cause fruit deformation and poor growth.

The application of the BCA or of the composition of the invention can be useful to protect at the same time kiwifruit trees from the infections caused by the three pseudomonades described above.

In the meaning of the invention a further plant disease of bacterial or fungal aetiology is caused by *P. syringae* pv. *mors-prunorum* or *morsprunorum* (Wormald 1931) Young, Dye, and Wilkie (1978) having the following taxonomic position: Bacteria: Gracilicutes the common name of the disease caused by this Gram-bacterium being Bacterial canker of Stone Fruit (English).

It has been reported in the EPPO Region, and attacks *Prunus* spp.; in particular, the fruit crops apricots, cherries, peaches causing
Bacterial canker of stone fruit.

An important, emerging plant disease called Bacterial Decline of apricot trees (particularly susceptible are vigorous cultivars like "Aurora", "Gold Strike", etc.) has been shown by experimental evidence with a complex aetiology and can be associated to virulent strains belonging to the three bacterial species above (*P. syringae* pv. *morsprunorum,* pv. *syringae* and *P*. *viridiflava*).

Typical symptoms may appear on all the aerial parts of the tree; particularly severe are cankers on branches associated to heavy gum production. Heavy infections may lead to the destruction of whole trees.

The pathogen may be present in asymptomatic propagation materials, raising the problem of the marketing of latently infected materials. Early and late copper sprays may help to protect trees from infections but with a limited effectiveness.

Also in this case the BCA or composition of the invention can also be used for the treatment of propagation material.

In the meaning of the invention one of the plant diseases of bacterial or fungal aetiology is caused by *Pseudomonas syringae* pv. *persicae* (Prunier et al.) Young et al. Synonyms are: *Pseudomonas mors-prunorum* f.sp. *persicae* Prunier et al having the following taxonomic position: Bacteria: Gracilicutes, the common name of the disease caused by this Gram-bacterium being Bacterial dieback of peach (English); Déperissement bactérien du pecher (French).

The bacterium is a quarantine pest included in the EPPO A2 list.

It that has been reported in Europe (France) and Oceania and affects peaches, nectarines (*Prunus persica*) and *P. salicina.*

Typical symptoms of the disease are visible on shoots and branches (brown discolorations, bark lesions, bud necrosis, dieback, wilting, cankers), on leaves (necrotic spots, "shot hole" effect) and fruits (superficial necrotic spots). In the most severe cases, entire branches and even the whole tree may die. This is a serious disease especially on highly susceptible peach cultivars (Hale, Redwing).

*P. syringae* pv. *persicae* has an important epiphytic phase on leaves in autumn that constitutes the inoculums for infections and enters shoots through pseudo-wounds (leaf scars) and wounds (pruning, freezing). Pathogen's spread is mainly due to asymptomatic propagation material; phytosanitary measures must be followed (inspections in nurseries, production of disease-free nursery stock, etc..) to limit spread over short and long distances.

Control is mainly based on prophylactic measures and copper sprays during leaf fall seem to limit the disease in orchards.

In the meaning of the invention one of the plant diseases of bacterial or fungal aetiology is caused by *Pectobacterium carotovorum* subsp. *carotovorum* (Jones) Hauben et al. Synomim: *Erwinia carotovora* subsp. *carotovora* (Jones) Bergey, Harrison et al having the following taxonomic position: Bacteria: Gracilicutes, the common name of the disease caused by this Gram-bacterium being Bacterial soft rot (English).

The bacterium is not a quarantine pest, has a worldwide distribution, and affects numerous horticultural (vegetables such as squash, pepper, fennel, celery, carrot, potato, etc...) and ornamental species.

The most typical symptom is the softening of the fleshy organs of the plant which turn brown and smell rotten. Heavy infections, occurring in the glasshouse, in the field and in store, may lead to very serious losses. This opportunistic pathogen survives in soils, in irrigation waters containing organic matter, in infected debris and in the rhizosphere of many cultivated and wild plant species. Wounds, usually associated to several stress events (heavy rains, high temperatures and humidity, excessive nitrogen manuring) are the major sites of bacterial penetration. Wind, aerosols, insects and man are important factors for pathogen and disease spread.

Control is mainly based on prophylactic measures, based on proper farming practices (e.g.: fertilization with low nitrogen and high potassium, crop rotation and field sanitation, insect control, good soil drainage) and copper sprays (every 7-10 days) when the disease appears may help to limit the capacity of the pathogen to readily colonize the host.

In a further meaning of the invention, one of the plant diseases of bacterial or fungal aetiology is caused by *Fusarium oxysporum* f. spp., a species belonging to the group of Mitosporic fungi, which comprises a vast number of *formae speciales* such as:
*Fusarium oxysporum* f.sp. *albedinis; Fusarium oxysporum* f.sp. *asparagi; Fusarium oxysporum* f.sp. *batatas; Fusarium oxysporum* f.sp. *betae; Fusarium oxysporum* f.sp. *cannabis; Fusarium oxysporum* f.sp. *cepae; Fusarium oxysporum* f.sp. *ciceris; Fusarium oxysporum* f.sp. *citri; Fusarium oxysporum* f.sp. *coffea; Fusarium oxysporum* f.sp. *cubense; Fusarium oxysporum* f.sp. *dianthi; Fusarium oxysporum* f.sp. *lentis; Fusarium oxysporum* f.sp. *lini; Fusarium oxysporum* f.sp. *lycopersici; Fusarium oxysporum* f.sp. *melonis; Fusarium oxysporum* f.sp. *nicotianae; Fusarium oxysporum* f.sp. *niveum; Fusarium oxysporum* f.sp. *passiflorae; Fusarium oxysporum* f.sp. *phaseoli; Fusarium oxysporum* f.sp. *pisi; Fusarium oxysporum* f.sp. *radicis-lycopersici; Fusarium oxysporum* f.sp. *ricini; Fusarium oxysporum* f.sp. *tulipae; Fusarium oxysporum* f.sp. *vasinfectum.*
The fungus, has a worldwide distribution and is detrimental for the agriculture due also to its wide range of targets. It attacks, causing severe losses, most vegetables and flowers (asparagus, bean, onion, pea, tomato, etc...), several field crops such as cotton and tobacco, plantation crops such as banana, plantain, coffee, and sugarcane, and a few shade trees. Great losses may be caused by the disease ("*Fusarium* wilt"), especially on susceptible varieties and under favourable weather conditions (soil and air temperatures rather high during much of the season). This vascular disease damages plants by causing stunting of plants, which soon wilt and finally die; when plants are infected at the seedling stage, they usually wilt and die soon after the appearance of the first symptoms. Roots also become infected, and after an initial period of stunting the smaller roots rot. This mitosporic fungus produces three kinds of asexual spores (micro-macroconidia, chlamydospores) and can survive between crops in infected plant debris in the soil also for long. Occasionally the fungus may reach the fruit of infected plants and penetrate or contaminate the seed. *Fusarium* spreads over short distances by means of water and contaminated farm equipment, and over long distances primarily in infected transplants or in the soil carried with them. Control is mainly based on the use of resistant varieties (when available), seedbed sterilization and crop rotation, use of healthy seed and transplants, hot-water treatment of seed, solarisation of field soil, and biological control using antagonistic fungi (*Trichoderma* spp) or beneficial siderophore-producing bacteria (*Pseudomonas* spp.).

When the diseases to be treated are among the diseases listed above, the skilled person will, by way of example, according to the invention the bacterium can be directly applied on the plant or part thereof to be treated.

In all the exemplified cases, the skilled person will know which parts of the plant have to be treated with the BCA or with the composition of the invention, and when to apply the antagonist in order to protect the trees from infection (e.g.: at least three spray applications per year on the whole tree).

According to the invention, the bacterium can be stored in a freeze-dried form, or as a powder such as a "Talc-based bio formulation" (an alginate mixture combined with sterilized talc as described by Ozaktan H., T. Bora 2006: "Studies on biological fire blight with some antagonistic bacteria" Acta Horticulturae 704: 337-340), as a granulate, and can be used as such on the plant or part thereof or can be resuspended in a suitable liquid such as brine, water, growth medium or other liquids known by the skilled person.

The bacterium can also be suspended in a suitable biopolymer gel by way of non limiting example according to the embodiments described in WO 0215702; and by Stockwell V.O., K.B. Johnson, Von W. Johnson 2006: "Colonization of flowers by Pseudomonas fluorescens A506 formulated in a biopolymer gel" Acta Horticulturae 704: 293-299).

The skilled person will know how to resuspend the bacterium prior to use it directly on the plant or on the plant part.

In one embodiment, the bacterium can be comprised in a composition that comprising also a suitable carrier for the bacterium (e.g. water, brine, growth medium, polymer gel, etc.), the composition optionally further comprising one or more additional compound suitable for aiding the colonisation of the plant by the bacterium of the invention (e.g. sodium alginates, amino acids, iron chelates).

According to the invention, the composition can also comprise for concomitant or subsequent application, one or more additional compound suitable for aiding the plant reactivity/recovery (e.g.: proper fertilization) and/or one or more additional compound commonly used as pesticide (copper-based products) and/or one or more other active additional compounds such (prohexadione-Ca, acybenzolar-S-methyl, phosethyl-AI) known as inducers or "elicitors" of disease resistance responses. When using compounds with a bactericidal effect, the BCA application should be done at different time intervals. The composition can be prepared for the concomitant or subsequent application of the bacterium and of the known pesticides. The control protocol can be such that a first application of a pesticide is made and a subsequent application/s of the bacterium is then carried out allowing a better antagonism by the bacterium of the invention against the pathogens.

In one embodiment the bacterium or a bacterial aliquot is suspended in a suitable carrier as indicated above and the BCA or the composition obtained as indicated above can be directly applied on the aerial organs of the plant, on propagation materials (seeds, tubers, etc...), in soils and on the root apparatus of plantlets before transplanting.

Conveniently, the bacterium can be stored in a freeze-dried or lyophilised form, or as a powder or granulate and the same can be resuspended in a suitable liquid or polymer gel known by the skilled person.

The invention hence also provides a kit for the prevention and/or treatment of plant diseases of bacterial or fungal aetiology, the kit comprising one or more aliquot of the bacterium as defined above and one or more aliquot of a vehicle and/or of a composition further comprising one or more of the additional compounds listed above, wherein either the vehicle aliquot or the composition aliquot can be used for the resuspension of the bacterial aliquot prior to use. When a composition for subsequent application is made, the skilled person will know, without use of inventive skill and without need of a further teaching in the present specification, which pesticides can be used for preparing the composition of the invention or, when a kit is prepared, which pesticides can be comprised in the aliquots of said composition further comprising one or more additional compound as described above.

By way of non-limiting example the pesticide can be selected from commonly used pesticides such as the ones described above.

In a preferred embodiment, the pesticide will be at a concentration below the concentration at which the same is normally used for the same purpose in the state of the art.

In an embodiment of the invention, the BCA can be a derived bacterium as described above, wherein said bacterium has been transformed or mutated so to be resistant to a specific antibiotic in order to allow the co-application of the antibiotic and the BCA when a strong treatment is advisable.

It is obvious that the invention refers also to a method for the preparation of the composition of the invention comprising the step of mixing the bacterium of the invention with one or more of a suitable carrier and/or a suitable bacterial nutrient and/or a suitable plant nutrient and/or a suitable pesticide.

The invention also refers to a method for the preparation of kit of the invention comprising the step of aliquoting the bacterium of the invention and optionally submitting the aliquots thus obtained to a storage step such as freeze drying, powdering, granulating or introducing the bacteria in a biopolymer gel by means of standard techniques as described above and preparing one or more aliquots of a suitable vehicle for the resuspension of the bacterial aliquot as defined above and/or preparing aliquots of a composition comprising one or more of the additional compounds listed above in which the bacterial aliquot can be resuspended or for application to the plant together, before or after the application of the bacterial aliquot as such or resuspended in said suitable vehicle.

The skilled person will know the suitable components of the composition from the common knowledge in the specific field, however, by way of non limiting example, the ingredients of the composition can be selected from any of the ingredients mentioned above.

The composition of the invention can be in any suitable form known to the skilled person for the treatment of plants or parts thereof, by non limiting way of example the composition can be in the form of a freeze-dried, granulate, powder, liquid, nebulised, tablet, capsule, polymer gel.

The concentration of the bacterium of the invention in the composition can be approx. 10¹¹ CFU/g of weight of the composition.

The invention further provides a method of treating a plant or a part thereof for the prevention and/or the treatment of plant diseases of bacterial or fungal aetiology comprising applying to a plant or a part thereof the bacterium or the composition or aliquots of the kit as previously described.

According to the method of the invention the application can be repeated from one to more times in subsequent periods of time.

When a composition for subsequent administration of pesticide and bacterium of the invention is used, the protocol of application will comprise a first step of application of suitable pesticides and a second step of application of the bacterium of the invention.

In one embodiment of the invention said, plant diseases of bacterial or fungal aetiology are selected from one or more diseases caused by *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae pv. actinidiae, Pseudomonas syringae pv. persicae; Pseudomonas viridiflava, Agrobacterium vitis, Pectobacterium carotovorum* subsp. *carotovorum* and *Fusarium oxysporum* f. spp.

The skilled person will know, when a subsequent application protocol is used, which pesticides will be suitable for carrying out the method of the invention.

An example of a possible application protocol of the BCA composition (approx. 10¹¹ CFU/g) against fire blight caused by *E. amylovora,* according to the most critical phases of the disease on different hosts and to the biological aspects of the pathogen under different environmental conditions, could be the following: a first application in Spring during bloom to prevent flower infections, followed by one or more applications during the vegetative period to protect shoots, fruits and second blossoms. The BCA applications could be integrated with the chemicals listed above, paying attention to avoid the use of antibiotics and/or other molecules with a bactericidal effect when the bacterium is applied simultaneously to the additional compounds. In case antibiotics or bactericidal compounds are used, a subsequent application protocol can be followed, wherein the application of the bacterium or of the composition of the invention is carried out preferably after the antibiotic or bactericidal compounds are used. The skilled person will know the duration of the effect of the antibiotic or of the bactericidal compound in general and will apply the bacterium or the composition of the invention when the effect of the aforementioned compounds is expired or strongly diminished. The composition for subsequent application or the kit for the same may be used to carry out the method described above. To develop a cost-effective control programme that works consistently, additional predictive programmes for forecasting fire blight disease in apples and pears can be used.

Another example of a possible application protocol of the BCA composition (approx. 10¹¹ CFU/g) against bacterial spot/canker of stone fruit crops caused by *Xanthomonas arboricola* pv. *pruni,* could be designed to prevent blossom, leaf, fruit, shoot/twig infections and late infections of shoots through leaf scars just before and during leaf fall in the autumn which constitute the primary inoculums source for the following Spring. Copper-based sprays could be advantageously done during the dormant period.

In one embodiment of the invention, a biotic vector can be used for the delivery of the biocontrol bacterium to the plants or parts thereof of interest.

It is well known that various insects preferably live, feed, reproduce on specific plants, the insect can hence be used as a living biotic vector for delivering the bacterium in the field on the plants or parts thereof of interest.

By way of non limiting example, pronubial hymenoptera such as, e.g. *Apidae* and *Megachilidae,* can be used as suitable biotic vectors in an embodiment of this invention.

The vectors can be put in contact with the bacterium of the invention (by way of example in the form of a high density living colonies preparation such as freeze-dried cells at a concentration of approx. 10¹¹ CFU/g) and then allowed to fly on the plants of interest so to deliver on said plants the bacterium of the invention, thus allowing the bacterium to grow on the plant or on a part thereof.

The following examples are experimental examples of tests carried out with the bacterium of the invention.

### EXAMPLES

### Assays on flowers of Actinidia spp.

Population dynamics of the antagonist bacterium: female flowers (5 flowers X survey/thesis) of A. *chinensis* 'Hort16A' Zespri Gold *A. deliciosa* 'Hayward' still closed were taken and put into 1.5 ml Eppendorf tubes containing 1 ml of sterile deionised water. On the next day, a suspension of a rifampicin-resistant mutant (refraction) of strain *P. fluorescens* BB20 deposit number under the Budapest treaty DSM 25556 (approx. 10⁸ colony forming units, CFU/ml) was nebulised on open flowers. The flowers were washed individually in 3 ml of 10 mM MgSO₄, decimal dilutions of the washing liquid were then made, and all dilutions were then seeded on Petri dishes containing KB + rifampicin (100ppm) by deposing on the substrate 10µl drops. The plates were then incubated at 27 °C for 24 hours and bacterial colonies were counted and referred to millilitres of washing. The measurements were made on flowers 1, 24, 48, 72 and 96 hours after application of antagonistic bacteria.

Population dynamics of *P. syringae* pv. *actinidiae* compared with the antagonist bacterium: female flowers (5 flowers X survey/thesis) of *A. chinensis* 'Hort16A' Zespri Gold still closed were taken and put into 1.5 ml Eppendorf tubes containing 1 ml of sterile deionised water. On the next day, a suspension of the bacterium of the invention BB20 deposit number under the Budapest treaty DSM 25556 (ca.10⁸CFU/ml) or water (control flowers) was nebulised on open flowers. After 24 hours, the flowers were nebulised with a suspension (ca.10⁶CFU/ml) of *P*. *syringae.* pv. *actinidiae* strain CRA-FRU 3.1Rif^{r}. The flowers were washed individually in 3ml 10mM diMgSO₄ decimal dilutions of the washing liquid were then made, and all dilutions were then seeded on NSA + rifampicin (100ppm) to monitor the population of the pathogen strain and on KB to detect the same time also the population of the antagonist strain BB20 deposit number under the Budapest treaty DSM 25556, by depositing 10µl droplets on both substrates. The NSA + rifampicin plates and the KB plates were incubated at 27 °C for 24 hours, after which, the bacterial colonies were counted and referred to millilitres of washing. The measurements were made on flowers 1, 24, 48, hours nebulisation, while measurements on the antagonist population were carried out up to 96 hours after its nebulisation.

*In vitro* assays, with respect to *in vitro* inhibition, were carried out according to Vanneste et al., 1992 protocol.

The results of the antagonistic activity tests carried out with the bacterium of the invention are reported in Table 1 below.

**Table 1 shows the in vitro inhibitory activity of the BB20 bacterium deposit number under the Budapest treaty DSM 25556 vs. various pathogenic bacterial strains.**

| | **Ea** | **Av** | **Rs** | **Xap** | **Pcc** | **Pss** | **Psa** |
|---|---|---|---|---|---|---|---|
| **G20** | Pos. | Pos. | Pos. | Pos. | Pos. | Pos. | Pos. |

| | | | | | | | |
|---|---|---|---|---|---|---|---|
| Ea: *Erwinia amylovora* Av: *Agrobacterium vitis* Rs: *Ralstonia solanacearum* Xap: *Xanthomonas arboricola* pv. *pruni* Pcc: *Pectobacterium carotovorum* subsp. *carotovorum* Pss: *Pseudomonas syringae* pv. *syringae* Psa: *Pseudomonas syringae* pv. *actinidiae* | | | | | | | |

Preliminary unpublished data for the preparation of the biopolymer gel showed that a water suspension of the freeze dried bacterial cells (approx. 10⁸ CFU/ml) amended with sodium-alginate (0.5%) and glycerol (0.4%) improved the survival of the BCA on pear leaves (approx. 2 orders of magnitude) with respect to the fresh and/or the freeze dried culture.

## Claims

1. A *P*. *fluorescens* bacterium having deposit number under the Budapest treaty DSM 25556 or a *P. fluorescens* bacterium deriving from the bacterium having the deposit number DSM 25556 and maintaining all of the antipathogenic activities thereof for the prevention and/or treatment of plant diseases of bacterial or fungal aetiology.

2. The *P*. *fluorescens* bacterium according to claim 1 wherein said antipathogenic activities are
presence of *in vitro* inhibitory activity on solid substrate at least against: *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae pv. actinidiae, Pseudomonas syringae pv. persicae; Pseudomonas viridiflava, Agrobacterium vitis, Pectobacterium carotovorum* subsp. *carotovorum;* and
presence of an *vitro* inhibitory activity on solid medium of the miceliar development of the fungus *Fusarium oxysporum* f. spp.

3. The bacterium of any one of claims 1 or 2 wherein said plant diseases of bacterial or fungal aetiology are selected from one or more diseases caused by *Erwinia spp., Pseudomonas spp., Ralstonia spp., Agrobacterium spp., Pectobacterium spp., Xanthomonas spp. and Fusarium spp.*

4. The bacterium of claim 3 wherein said plant diseases of bacterial or fungal aetiology are selected from one or more diseases caused by *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae pv. actinidiae, Pseudomonas syringae pv. persicae; Pseudomonas viridiflava, Agrobacterium vitis, Pectobacterium carotovorum* subsp. *carotovorum; Fusarium oxysporum f. spp.* infections.

5. The bacterium of any one of claims 1 to 4 in freeze-dried, powder, granulate, gel, liquid suspension form.

6. A composition comprising the bacterium of any one of claims 1 to 5 and a suitable carrier.

7. The composition of claim 6 further comprising one or more additional compounds aiding the colonisation of the plant by the bacterium of any one of claims 1 to 5 and/or one or more additional compounds aiding the plant reactivity/recovery and/or one or more additional compounds having pesticide properties and/or one or more additional compounds selected from inducers or elicitors of disease resistance responses in plants.

8. The composition of claim 6 or 7 in a freeze-dried, granulate, powder, liquid suspension, nebulised, tablet, capsule, gel, biopolymer gel, cream, ointment, paste, emulsion, medicated device form.

9. A kit of parts comprising one or more aliquot of the bacterium of any one of claims 1 to 5, and one or more aliquot of a suitable vehicle and/or of a composition further comprising one or more of the additional compounds as defined in claim 7.

10. The kit of claim 9 wherein the kit comprises one or more aliquot of the bacterium of claims 1 to 5, one or more aliquots of a suitable vehicle and one or more aliquots of a composition comprising one or more compounds having pesticide properties including bactericidal properties.

11. A method of treating a plant or a part thereof for the prevention and/or the treatment of plant diseases of bacterial or fungal aetiology comprising applying to a plant or a part thereof the bacterium or the composition of any one of claims 1 to 7.

12. A method of treating a plant or a part thereof for the prevention and/or the treatment of plant diseases of bacterial or fungal aetiology comprising applying subsequently to a plant or a part thereof a composition comprising one or more compounds having pesticide properties including bactericidal properties, and the bacterium or the composition of any one of claims from 1 to 8.

13. The method of any one of claims 11 or 12 wherein said diseases of bacterial and/or fungal aetiology are plant diseases selected from one or more diseases caused by *Erwinia spp., Pseudomonas spp., Ralstonia spp., Agrobacterium spp., Pectobacterium spp., Xanthomonas spp.* and *Fusarium spp.*

14. The method of claim 13 wherein said plant diseases of bacterial or fungal aetiology are selected from one or more diseases caused by *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae pv. actinidiae, Pseudomonas syringae pv. persicae; Pseudomonas viridiflava, Agrobacterium vitis, Pectobacterium carotovorum* subsp. *carotovorum; Fusarium oxysporum f. spp.* infections.

15. A method of treating a plant or a part thereof for the prevention and/or the treatment of plant diseases of bacterial and/or fungal aetiology comprising:
- putting in contact a living host normally living on or normally present on said plant or part thereof with the living bacterium of any one of claims 1 to 5 and
- allowing said host to contact said plant or part thereof thus allowing said bacterium to grow on said plant or part thereof

## Patentansprüche

1. *P*. *fluorescens*-Bakterium mit der Hinterlegungsnummer DSM 25556 nach dem Budapester Vertrag, oder *P*. *fluorescens*-Bakterium, das sich von dem Bakterium mit der Hinterlegungsnummer DSM 25556 herleitet und alle seine antipathogenen Aktivitäten zur Prävention und/oder Behandlung von Pflanzenkrankheiten bakteriellen oder mykotischen Ursprungs beibehält.

2. *P*. *fluorescens*-Bakterium gemäß Anspruch 1, wobei die antipathogenen Aktivitäten die folgenden sind:
Vorkommen einer *In-vitro*-Hemmaktivität auf festem Substrat mindestens gegen *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae pv. actinidiae, Pseudomonas syringae pv. persicae; Pseudomonas viridiflava, Agrobacterium vitis, Pectobacterium carotovorum* subsp. *carotovorum;* und
Vorkommen einer *In-vitro*-Hemmaktivität auf festem Medium der mizellaren Entwicklung des Pilzes *Fusarium oxysporum* f. spp.

3. Bakterium gemäß einem der Ansprüche 1 oder 2, wobei die Pflanzenkrankheiten bakteriellen oder mykotischen Ursprungs aus einer oder mehreren Krankheiten ausgewählt werden, die durch *Erwinia spp., Pseudomonas spp., Ralstonia spp., Agrobakterium spp., Pectobacterium spp., Xanthomonas spp.* und *Fusarium spp.* verursacht werden.

4. Bakterium gemäß Anspruch 3, wobei die Pflanzenkrankheiten bakteriellen oder mykotischen Ursprungs aus einer oder mehreren Krankheiten ausgewählt werden, die durch Infektionen mit *Xanthomonas arboricola pv. pruni, Ralstonia solanacearum, Erwinia amylovora, Pseudomonas syringae pv. syringae, Pseudomonas syringae pv. morsprunorum, Pseudomonas syringae pv. actinidiae, Psudomonas syringae pv. persicae; Pseudomonas viridiflava, Agrobacterium vitis, Pectobacterium carotovorum* subsp. *carotovorum; Fusarium oxysprorum f. spp.* verursacht werden.

5. Bakterium gemäß einem der Ansprüche 1 bis 4 in Form eines Lyophilisats, eines Pulvers, eines Granulats, eines Gels, einer flüssigen Suspension.

6. Zusammensetzung, die das Bakterium gemäß einem der Ansprüche 1 bis 5 und einen geeigneten Träger umfasst.

7. Zusammensetzung gemäß Anspruch 6, die weiterhin umfasst: eine oder mehrere zusätzliche Verbindungen, die die Besiedlung der Pflanze durch das Bakterium gemäß einem der Ansprüche 1 bis 5 unterstützen, und/oder eine oder mehrere zusätzliche Verbindungen, die die Reaktionsfähigkeit/Erholung der Pflanze unterstützen, und/oder eine oder mehrere zusätzliche Verbindungen, die über Pestizideigenschaften verfügen, und/oder eine oder mehrere zusätzliche Verbindungen, die aus Induktoren oder Auslösern von pflanzlichen Krankheitsresistenzreaktionen ausgewählt werden.

8. Zusammensetzung gemäß Anspruch 6 oder 7 in Form eines Lyophilisats, eines Granulats, eines Pulvers, einer flüssigen Suspension, einer Vernebelung, einer Tablette, einer Kapsel, eines Gels, eines Biopolymer-Gels, einer Creme, einer Salbe, einer Paste, einer Emulsion, einer medizinischen Vorrichtung.

9. Kit aus Teilen, das umfasst: mindestens eine Aliquote des Bakteriums gemäß einem der Ansprüche 1 bis 5 und mindestens eine Aliquote eines geeigneten Trägers und/oder eine Zusammensetzung, die weiterhin mindestens eine der zusätzlichen Verbindungen gemäß Anspruch 7 umfasst.

10. Kit gemäß Anspruch 9, wobei das Kit umfasst: mindestens eine Aliquote des Bakteriums gemäß den Ansprüchen 1 bis 5, mindestens eine Aliquote eines geeigneten Trägers und mindestens eine Aliquote einer Zusammensetzung, die mindestens eine Zusammensetzung umfasst, die über Pestizideigenschaften, die bakterizide Eigenschaften umfassen, verfügt.

11. Verfahren zum Behandeln einer Pflanze oder eines Teils davon zur Prävention und/oder Behandlung von Pflanzenkrankheiten bakteriellen oder mykotischen Ursprungs, das umfasst: Aufbringen des Bakteriums oder der Zusammensetzung gemäß einem der Ansprüche 1 bis 7 auf eine Pflanze oder einem Teil davon.

12. Verfahren zum Behandeln einer Pflanze oder eines Teils davon zur Prävention und/oder Behandlung von Pflanzenkrankheiten bakteriellen oder mykotischen Ursprungs, das umfasst: nachfolgendes Aufbringen einer Zusammensetzung auf die Pflanze oder einem Teil davon, die mindestens eine Verbindung umfasst, die über Pestizideigenschaften, die bakterizide Eigenschaften umfassen, verfügt, und des Bakteriums oder der Zusammensetzung gemäß einem der Ansprüche 1 bis 8.

13. Verfahren gemäß einem der Ansprüche 11 oder 12, wobei die Krankheiten bakteriellen und/oder mykotischen Ursprungs Pflanzenkrankheiten sind, die aus einer oder mehreren Krankheiten ausgewählt werden, die durch *Erwinia spp., Pseudomonas spp., Ralstonia spp., Agrobacterium spp., Pectobacterium spp., Xanthomonas spp.* und *Fusarium spp.* verursacht werden.

14. Verfahren gemäß Anspruch 13, wobei die Pflanzenkrankheiten bakteriellen oder mykotischen Ursprungs aus einer oder mehreren Krankheiten ausgewählt werden, die durch Infektionen mit *Xanthomonas arboricola pv. pruni, Ralstonia solanacearum, Erwinia amylovora, Pseudomonas syringae pv. syringae, Pseudomonas syringae pv. morsprunorum, Pseudomonas syringae pv. actinidiae, Pseudomonas syringae pr. persicae; Pseudomonas viridiflava, Agrobacterium vitis, Pectobacterium carotovorum* subsp. *carotovorum; Fusarium oxysporum f. spp.* verursacht werden.

15. Verfahren zum Behandeln einer Pflanze oder eines Teils davon zur Prävention und/oder Behandlung von Pflanzenkrankheiten bakteriellen und/oder mykotischen Ursprungs, das umfasst:
- einen lebenden Wirt, der normalerweise auf der Pflanze oder Teilen davon lebt oder darauf vorkommt, mit dem lebenden Bakterium gemäß einem der Ansprüche 1 bis 5 in Kontakt zu bringen und
- zu gewährleisten, dass der Wirt Kontakt mit der Pflanze oder einem Teil davon aufnimmt, wodurch gewährleistet wird, dass das Bakterium auf der Pflanze oder einem Teil davon wächst

## Revendications

1. Bactérie *P. fluorescens* ayant le numéro de dépôt sous le Traité de Budapest DSM 25556 ou bactérie *P. fluorescens* dérivant de la bactérie ayant le numéro de dépôt DSM 25556 et conservant la totalité de ses activités antipathogéniques pour la prévention et/ou le traitement de maladies végétales d'étiologie bactérienne ou fongique.

2. Bactérie *P. fluorescens* selon la revendication 1, dans laquelle lesdites activités antipathogéniques sont
la présence d'une activité inhibitrice *in vitro* sur un substrat solide au moins contre : *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae* pv. *actinidiae, Pseudomonas syringae* pv. *persicae ; Pseudomonas viridiflava, Agrobacterium vitis, Pectobacterium carotovorum* subsp. *carotovorum* ; et
la présence d'une activité inhibitrice *in vitro* sur un milieu solide du développement micellaire du champignon *Fusarium oxysporum* f. spp.

3. Bactérie selon l'une quelconque des revendications 1 ou 2, où lesdites maladies végétales d'étiologie bactérienne ou fongique sont choisies parmi une ou plusieurs maladies provoquées par *Erwinia spp., Pseudomonas spp., Ralstonia spp., Agrobacterium spp., Pectobacterium spp., Xanthomonas spp.,* et *Fusarium spp.*

4. Bactérie selon la revendication 3, où lesdites maladies végétales d'étiologie bactérienne ou fongique sont choisies parmi une ou plusieurs maladies provoquées par des infections à *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae* pv. *actinidiae, Pseudomonas syringae* pv. *persicae ; Pseudomonas viridiflava, Agrobacterium vitis, Pectobacterium carotovorum* subsp. *carotovorum ; Fusarium oxysporum f. spp.*

5. Bactérie selon l'une quelconque des revendications 1 à 4 sous forme cryodesséchée, de poudre, de granulat, de gel, de suspension liquide.

6. Composition comprenant la bactérie selon l'une quelconque des revendications 1 à 5 et un support approprié.

7. Composition selon la revendication 6 comprenant en outre un ou plusieurs composés supplémentaires aidant la colonisation de la plante par la bactérie selon l'une quelconque des revendications 1 à 5 et/ou un ou plusieurs composés supplémentaires aidant la réactivité/récupération de la plante et/ou un ou plusieurs composés supplémentaires possédant des propriétés pesticides et/ou un ou plusieurs composés supplémentaires choisis parmi des inducteurs et des déclencheurs de réponses de résistance aux maladies dans les plantes.

8. Composition selon la revendication 6 ou 7 sous une forme cryodesséchée, de granulat, de poudre, de suspension liquide, nébulisée, de comprimé, de capsule, de gel, de gel de biopolymère, de crème, de pommade, de pâte, d'émulsion, de dispositif médicamenteux.

9. Kit de parties comprenant une ou plusieurs aliquotes de la bactérie selon l'une quelconque des revendications 1 à 5, et une ou plusieurs aliquotes d'un véhicule approprié et/ou d'une composition comprenant en outre un ou plusieurs des composés supplémentaires tels que définis dans la revendication 7.

10. Kit selon la revendication 9, dans lequel le kit comprend une ou plusieurs aliquotes de la bactérie selon les revendications 1 à 5, une ou plusieurs aliquotes d'un véhicule approprié et une ou plusieurs aliquotes d'une composition comprenant un ou plusieurs composés possédant des propriétés pesticides y compris des propriétés bactéricides.

11. Procédé de traitement d'une plante ou de l'une de ses parties pour la prévention et/ou le traitement de maladies végétales d'étiologie bactérienne ou fongique comprenant l'application à une plante ou à l'une de ses parties de la bactérie ou de la composition selon l'une quelconque des revendications 1 à 7.

12. Procédé de traitement d'une plante ou de l'une de ses parties pour la prévention et/ou le traitement de maladies végétales d'étiologie bactérienne ou fongique comprenant l'application subséquemment à une plante ou à l'une de ses parties d'une composition comprenant un ou plusieurs composés possédant des propriétés pesticides y compris des propriétés bactéricides, et de la bactérie ou de la composition selon l'une quelconque des revendications 1 à 8.

13. Procédé selon l'une quelconque des revendications 11 ou 12, où lesdites maladies d'étiologie bactérienne et/ou fongiques sont des maladies végétales choisies parmi une ou plusieurs maladies provoquées par *Erwinia spp., Pseudomonas spp., Ralstonia spp., Agrobacterium spp., Pectobacterium spp., Xanthomonas spp.* et *Fusarium spp.*

14. Procédé selon la revendication 13 où lesdites maladies végétales d'étiologie bactérienne ou fongique sont choisies parmi une ou plusieurs maladies provoquées par des infections à *Xanthomonas arboricola* pv. *pruni, Ralstonia solanacearum, Erwinia amylovora, Pseudomonas syringae* pv. *syringae, Pseudomonas syringae* pv. *morsprunorum, Pseudomonas syringae* pv. *actinidiae, Pseudomonas syringae* pv. *persicae ; Pseudomonas viridiflava, Agrobacterium vitis, Pectobacterium carotovorum* subsp. *carotovorum ; Fusarium oxysporum f. spp.*

15. Procédé de traitement d'une plante ou de l'une de ses parties pour la prévention et/ou le traitement de maladies végétales d'étiologie bactérienne et/ou fongique comprenant :
- la mise en contact d'un hôte vivant, vivant normalement sur ou normalement présent sur ladite plante ou l'une de ses parties avec la bactérie vivante selon l'une quelconque des revendications 1 à 5 et
- l'étape permettant au dit hôte d'entrer en contact avec ladite plante ou l'une de ses parties permettant ainsi à ladite bactérie de se développer sur ladite plante ou l'une de ses parties.
